# FASCICULE DE BREVET EUROPEEN

(11) **EP 0 880 979 B1**
(45) Date de publication et mention de la délivrance du brevet: **21.12.2005**
(21) Numéro de dépôt: 98401161.9
(22) Date de dépôt: 15.05.1998
(51) Int. Cl.: A61N 1/362

(54) **Stimulateur cardiaque, défibrillateur et/ou cardioverteur à réduction des épisodes d'arythmie auriculaire**
Herzschrittmacher, Defibrillator und/oder Cardioverter zur Verringerung von Vorhofsarrythmien
Atrial arrhythmia reducing pacemaker, defibrillator and/or cardioverter

(30) Priorité: 16.05.1997 FR 9706021
(43) Date de publication de la demande: 02.12.1998
(73) Titulaire: ELA MEDICAL (Société anonyme), 92541 Montrouge (FR)
(72) Inventeur: Bouhour, Anne, 92410 Ville d'Avray (FR); Limousin, Marcel, 75014 Paris (FR); Bonnet, Jean-Luc, 92120 Montrouge (FR)
(74) Mandataire: Dupuis-Latour, Dominique

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

Dans la suite, on s'intéressera principalement au traitement des troubles du rythme auriculaire par de tels appareils, qu'il s'agisse d'appareils "double chambre" qui recueillent et délivrent des signaux à la fois dans l'oreillette et le ventricule, ou d'appareils "simple chambre" recueillant et délivrant des signaux seulement dans l'oreillette.

La localisation auriculaire des troubles traités par l'invention n'est cependant pas limitative, et l'invention pourra être également appliquée, *mutetis mutandis,* au traitement de troubles du rythme ventriculaire, la cavité du myocarde considérée étant alors le ventricule au lieu de l'oreillette.

Le terme "troubles du rythme auriculaire" ou TdRA est un terme générique qui recouvre diverses arythmies auriculaires (épisodes d'accélération non physiologique du rythme) telles que tachycardie, fibrillation, *flutter,* etc., troubles tous caractérisés, à la détection, par un rythme auriculaire rapide.

Lorsque l'on détecte un TdRA, c'est-à-dire essentiellement lorsque le rythme auriculaire dépasse un niveau admissible, le stimulateur bascule en un mode dit de "désynchronisation" ou de "dissociation auriculo-ventriculaire" pour stimuler le ventricule indépendamment du rythme auriculaire détecté, puisque ce rythme excessif est considéré comme pathologique.

De façon générale, on a observé que les patients présentaient moins d'arythmies auriculaires quand ils étaient stimulés de façon permanente.

Une solution simple consisterait à stimuler en permanence l'oreillette ou les oreillettes à un rythme fixe très supérieur au rythme physiologique, mais elle serait toutefois néfaste, à la longue, à l'état clinique du patient.

Le US-A-4 419 996 décrit un dispositif susceptible de fonctionner selon deux modes différents, à savoir un premier mode normal, inhibé, convenant pour traiter les arythmies de type bradycardiques, et un second mode, dissocié, sous la commande d'un boîtier externe programmé de manière à traiter les arythmies de type tachycardiques. Le basculement du premier vers le second mode est obtenu en approchant un aimant, ce qui suppose que c'est le patient, ou un praticien, qui décide du passage d'un mode à l'autre. Le dispositif opère donc de manière non automatique et en fonction d'une appréciation personnelle du patient ou du praticien.

Le WO-A-95 09661 décrit un dispositif permettant la stimulation asynchrone de l'oreillette, stimulation qui est mise en oeuvre en fonction de l'évolution du rythme sinusal du patient et de l'information fournie par un capteur de l'activité du patient afin d'adapter le fonctionnement aux besoins physiologiques réels du patient. Ce dispositif présente cependant l'inconvénient de nécessiter une source d'information supplémentaire (le capteur d'activité) qui, en outre, n'est pas disponible en permanence, par exemple lorsque le patient est au repos.

Le but de la présente invention est de remédier à ces inconvénients en proposant un dispositif qui puisse assurer une stimulation quasi-permanente d'une cavité cardiaque, notamment de la cavité atriale, tout en conservant un rythme adapté aux besoins physiologiques du patient, c'est-à-dire un rythme très légèrement supérieur au rythme spontané sous-jacent en l'absence de stimulation, et ceci de manière autonome et automatique.

Le dispositif de l'invention est du type connu comprenant : des moyens de détection d'événements d'activité électrique spontanés dans une cavité cardiaque, de préférence l'oreillette ; des moyens pour délivrer une stimulation à ladite cavité cardiaque, commandés en fonction d'un intervalle d'échappement courant de telle sorte que, à chaque cycle cardiaque, une stimulation soit délivrée à la cavité si aucun événement spontané n'est détecté au bout d'un temps correspondant à l'écoulement de la période d'intervalle d'échappement depuis le dernier événement spontané ou la dernière stimulation dans la cavité ; des moyens de comptage du nombre d'événements stimulés successifs ; et des moyens de discrimination entre origine extrasystolique ou non des événements spontanés détectés dans la cavité.

Le WO-A-97/41922, qui fait partie de l'état de la technique au titre des art. 54(3) et 54(4) CBE, décrit un tel dispositif dans lequel l'intervalle d'échappement est modifiable à chaque cycle cardiaque de manière à réduire d'une première valeur programmée l'intervalle d'échappement courant après détection d'un événement spontané d'origine non extrasystolique, ou accroître d'une seconde valeur programmée l'intervalle d'échappement courant après délivrance d'un nombre prédéterminé de stimulations successives.

Selon l'invention, la première et/ou la seconde valeur programmée sont fonction du rythme cardiaque moyen précédant la réduction (ou, respectivement, l'accroissement) de l'intervalle d'échappement.

Selon diverses caractéristiques subsidiaires avantageuses :
- ledit nombre prédéterminé vaut 24 ;
- l'intervalle d'échappement est limité, dans le sens de l'accroissement, à une valeur plafond correspondant à la fréquence cardiaque de base programmée dans le dispositif;
- l'intervalle d'échappement est limité, dans le sens de la réduction, à une valeur plancher correspondant à la fréquence cardiaque maximale autorisée par le dispositif.

On va maintenant décrire un exemple de mise en oeuvre de l'invention, réalisable par exemple par une programmation appropriée du logiciel de commande du stimulateur.

On va tout d'abord donner un certain nombre de définitions utilisées dans la suite de la description.

"Onde ou événement P": recueil d'une activité spontanée ayant son origine dans l'oreillette

"Onde ou événement R" : recueil d'une activité spontanée ayant son origine dans le ventricule.
"Événement A" : stimulation délivrée à l'oreillette.
"Événement V" : stimulation délivrée au ventricule.
"Cycle cardiaque" : intervalle de temps séparant deux événements de même nature dans la même cavité, par exemple séparant deux ondes P, ou deux événements A.

"PP moyen" : intervalle moyen du rythme auriculaire, calculé par exemple sur 8 cycles cardiaques ne comprenant pas d'extrasystole.

"Intervalle d'échappement" (IE) : intervalle de temps, compté après une détection ou une stimulation dans une cavité donnée, à l'issue duquel une stimulation est délivrée à cette cavité si aucun événement spontané n'a été détecté dans cette même cavité. Pour l'oreillette, il s'agit de l'intervalle d'échappement auriculaire IEA.

"ESA": extrasystole auriculaire. Une onde P est définie comme une ESA si l'intervalle de temps séparant cette onde P du précédent événement auriculaire est inférieur à une fraction donnée de PP moyen.

"ESV" : extrasystole ventriculaire. On définit deux types d'ESV : une ESV de type 1 correspond à une détection ventriculaire non précédée d'un événement auriculaire dans un intervalle de temps compris par exemple entre 31 et 300 ms ; une ESV de type 2 correspond à une détection ventriculaire, précédée d'un événement auriculaire dans un intervalle de temps compris entre 31 et 300 ms, mais avec un changement du temps de conduction A-R par rapport au délai auriculo-ventriculaire (DAV) du cycle cardiaque précédent : DAV-AR > 31 ms).

Pour de plus amples détails sur les extrasystoles, on pourra notamment se référer au EP-A-0 550 342, au nom de ELA Médical, qui décrit un algorithme de détection et de traitement des ESV par une stimulation asynchrone de l'oreillette et une stimulation contrôlée du ventricule.

L'invention propose d'agir en continu sur l'intervalle d'échappement, notamment l'intervalle d'échappement auriculaire (IEA) si l'on s'intéresse à la cavité auriculaire, en adaptant la valeur de cet intervalle au cours des cycles cardiaques successifs en fonction des événements intervenus au cours de ces cycles.

Plus précisément, chaque fois que l'on détecte un événement auriculaire spontané (onde P) considéré comme sinusal et non comme une ESA, on diminue l'intervalle IEA courant d'une première valeur programmable ou "pente d'accélération" et on applique ce nouvel intervalle au cycle en cours.

Les techniques permettant de différencier une activité sinusale normale d'une ESA sont en elles-mêmes connues, par exemple d'après les FR-A-2 544 989 et EP-A-0 488 840, tous deux au nom de ELA Médical, qui décrivent un mode de traitement des ESA mis en oeuvre en particulier dans le stimulateur *Chorus II 6234* de cette même société.

Après un nombre donné de stimulations atriales consécutives (événements A), par exemple 24 stimulations consécutives, on allonge l'intervalle d'échappement IEA d'une seconde valeur programmable ou "pente de décélération".

Selon l'invention, on allonge ou on raccourcit l'intervalle d'échappement d'une valeur qui est elle-même fonction du rythme cardiaque moyen précédant la modification de l'intervalle d'échappement, par exemple une valeur qui est un pourcentage de la période cardiaque moyenne précédente.

Si l'on détecte une onde P qui est considérée par le dispositif comme étant une ESA, on conserve la valeur courante de l'intervalle d'échappement.

Ainsi, à la suite d'un ou plusieurs événements spontanés d'origine non extrasystolique, on va accélérer la fréquence de stimulation auriculaire jusqu'à disparition de ce rythme spontané.

Inversement, après un nombre prédéterminé de stimulations consécutives, on va allonger la période d'échappement jusqu'à recouvrer un événement spontané, ce qui permet de stimuler le coeur de façon presque permanente à un rythme légèrement supérieur à la fréquence spontanée sous-jacente du patient, en tenant compte des besoins physiologiques de ce dernier. Ces cycles peuvent comprendre des extrasystoles qui ne sont pas prises en compte et qui n'interrompent pas le comptage.

Bien entendu, l'excursion de l'intervalle d'échappement est limitée dans les deux sens : dans le sens de l'allongement, à la valeur de l'intervalle correspondant à la fréquence de base programmée du stimulateur, et dans le sens du raccourcissement à la valeur correspondant à la fréquence maximale programmée du stimulateur.

## Revendications

1. Un dispositif médical implantable actif, notamment un stimulateur cardiaque, défibrillateur et/ou cardioverteur, du type comprenant :
- des moyens de détection d'événements d'activité électrique spontanés dans une cavité cardiaque,
- des moyens pour délivrer une stimulation à ladite cavité cardiaque, commandés en fonction d'un intervalle d'échappement courant de telle sorte que, à chaque cycle cardiaque, une stimulation soit délivrée à la cavité si aucun événement spontané n'est détecté au bout d'un temps correspondant à l'écoulement de la période d'intervalle d'échappement depuis le dernier événement spontané ou la dernière stimulation dans la cavité,
- des moyens de comptage du nombre d'événements stimulés successifs, et
- des moyens de discrimination entre origine extrasystolique ou non des événements spontanés détectés dans la cavité, dans lequel l'intervalle d'échappement est modifiable à chaque cycle cardiaque de manière à :
- réduire d'une première valeur programmée l'intervalle d'échappement courant après détection d'un événement spontané d'origine non extrasystolique ladite première valeur programmée étant fonction du rythme cardiaque moyen précédant la réduction de l'intervalle d'échappement, ou
- accroître d'une seconde valeur programmée l'intervalle d'échappement courant après délivrance d'un nombre prédéterminé de stimulations successives ladite seconde valeur programmée étant fonction du rythme cardiaque moyen précédant l'accroissement de l'intervalle d'échappement.

2. Le dispositif médical de la revendication 1, dans lequel les moyens de détection détectent les événements spontanés atriaux.

3. Le dispositif médical de la revendication 1, dans lequel ledit nombre prédéterminé vaut 24.

4. Le dispositif médical de la revendication 1, dans lequel l'intervalle d'échappement est limité, dans le sens de l'accroissement, à une valeur plafond correspondant à la fréquence cardiaque de base programmée dans le dispositif.

5. Le dispositif médical de la revendication 1, dans lequel l'intervalle d'échappement est limité, dans le sens de la réduction, à une valeur plancher correspondant à la fréquence cardiaque maximale autorisée par le dispositif.

## Patentansprüche

1. Aktive, implantierbare medizinische Vorrichtung, insbesondere ein Herzschrittmacher, Defibrillator und/oder Kardioverter, von dem Typ, welcher umfasst:
- Mittel zur Erkennung spontaner elektrischer Aktivitätsereignisse in einer Herzkammer,
- Mittel zur Abgabe einer Stimulation an die Herzkammer, gesteuert in Abhängigkeit eines Auslassintervalls, verlaufend nach der Art, dass, bei jedem Herzzyklus, eine Stimulation an die Kavität abgegeben wird, wenn keinerlei spontanes Ereignis erkannt ist am Ende einer Zeit, die dem Ablauf der Periode des Auslassintervalls seit dem letzten spontanen Ereignis oder der letzten Simulation in der Kavität entspricht,
- Mittel zur Zählung der Anzahl von aufeinander folgenden stimulierken Ereignissen, und
- Mittel zur Unterscheidung zwischen extrasystolischem Ursprung und nicht extrasystolischem Ursprung von spontanen Ereignissen, die in der Kavität entdeckt wurden,
in welcher das Auslassintervall zu jedem Herzzyklus veränderbar ist, um:
- das aktuelle Auslassintervall um einen programmierten ersten Wert zu reduzieren nach Erkennung eines spontanen Ereignisses von nicht extrastystolischem Ursprung, wobei der programmierte Wert abhängig vom mittleren Herzrhythmus ist, der der Reduktion des Auslassintervalls vorhergeht, oder
- das aktuelle Auslassintervall um einen programmierten zweiten Wert zu erhöhen nach Abgeben einer vorherbestimmten Anzahl von aufeinander folgenden Stimulationen, wobei der zweite programmierte Wert abhängig vom mittleren Herzrhythmus ist, der der Erhöhung des Auslassintervalls vorhergeht.

2. Medizinische Vorrichtung nach Anspruch 1, in welcher die Mittel zur Erkennung die atrialen spontanen Ereignisse erkennen.

3. Medizinische Vorrichtung nach Anspruch 1, in welcher die vorherbestimmte Anzahl insofern 24 ist.

4. Medizinische Vorrichtung nach Anspruch 1, in welcher das Auslassintervall begrenzt ist, in der Richtung des Erhöhens, auf einen oberen Grenzwert, welcher der Basisherzfrequenz entspricht, die in der Vorrichtung programmiert ist.

5. Medizinische Vorrichtung nach Anspruch 1, in welcher das Auslassintervall begrenzt ist, in der Richtung der Reduzierung auf einen Mindestwerk, welcher der maximalen Herzfrequenz entspricht, der durch die Vorrichtung autorisiert ist.

## Claims

1. An active implantable medical device, in particular a cardiac pacemaker, defibrillator and/or cardioverter, of the type comprising:
- means for detecting events of spontaneous electrical activity in a cardiac cavity;
- means for delivering a stimulation to said cardiac cavity, controlled according to an actual escape interval in such a way that, at each cardiac cycle, a stimulation is delivered to the cavity if no spontaneous event is detected at the end of a time corresponding to the end of the period of escape interval since the last spontaneous event or the last stimulation in the cavity;
- means for counting the number of successive stimulated events; and
- means for discriminating between an extrasystolic origin and a non-extrasystolic origin of the spontaneous events detected in the cavity; wherein the escape interval may be modified at each cardiac cycle in a manner to
- reduce by a first programmed value the actual escape interval after detection of a spontaneous event of non-extrasystolic origin, the first programmed value being a function of the average cardiac rhythm preceding the reduction of the escape interval, or
- increase by a second programmed value the actual escape interval after delivery of a predetermined number of successive stimulations, the second programmed value being a function of the average cardiac rhythm preceding the increase of the escape interval.

2. The medical device of claim 1, wherein the detecting means detects the atrial spontaneous events.

3. The medical device of claim 1, wherein said predetermined number is 24.

4. The medical device of claim 1, wherein the escape interval is limited, in the sense of the increase, to a maximal value corresponding to the base cardiac stimulation frequency programmed in the device.

5. The medical device of claim 1, wherein the escape interval is limited, in the sense of the reduction, to a minimal value corresponding to the maximal cardiac frequency authorized by the device.
